(19) Europäisches Patentamt
European Patent Office
Office européen des brevets

(11) Veröffentlichungsnummer : **0 100 045**
**B1**

(12) **EUROPÄISCHE PATENTSCHRIFT**

(45) Veröffentlichungstag der Patentschrift : **21.01.87**

(21) Anmeldenummer : **83107013.1**

(22) Anmeldetag : **18.07.83**

(51) Int. Cl.[4] : **C 07 D285/08**, C 07 D417/12, A 01 N 43/82

(54) **Substituierte 3-Trichlormethyl-1,2,4-thiadiazol-5-yl-oxyessigsäureamide, Verfahren zu ihrer Herstellung und ihre Verwendung als Herbizide.**

(30) Priorität : **28.07.82 DE 3228131**

(43) Veröffentlichungstag der Anmeldung : **08.02.84 Patentblatt 84/06**

(45) Bekanntmachung des Hinweises auf die Patenterteilung : **21.01.87 Patentblatt 87/04**

(84) Benannte Vertragsstaaten : **BE CH DE FR GB IT LI NL**

(56) Entgegenhaltungen :
**DE-A- 3 038 636**
**US-A- 4 228 290**
**Die Akte enthält technische Angaben, die nach dem Eingang der Anmeldung eingereicht wurden und die nicht in dieser Patentschrift enthalten sind.**

(73) Patentinhaber : **BAYER AG**
**Konzernverwaltung RP Patentabteilung**
**D-5090 Leverkusen 1 Bayerwerk (DE)**

(72) Erfinder : **Förster, Heinz, Dr.**
**Am Eckbusch 47**
**D-5600 Wuppertal 1 (DE)**
Erfinder : **Diehr, Hans-Joachim, Dr.**
**Höhe 35**
**D-5600 Wuppertal 11 (DE)**
Erfinder : **Stetter, Jörg, Dr.**
**Gellertweg 4**
**D-5600 Wuppertal 1 (DE)**
Erfinder : **Eue, Ludwig, Dr.**
**Paul-Klee-Strasse 36**
**D-5090 Leverkusen 1 (DE)**
Erfinder : **Schmidt, Robert R., Dr.**
**Im Waldwinkel 110**
**D-5060 Bergisch-Gladbach 2 (DE)**

**EP 0 100 045 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue substituierte 3-Trichlormethyl-1,2,4-thiadiazol-5-yl-oxyessigsäureamide, ein Verfahren zu ihrer Herstellung und ihre Verwendung als Herbizide.

Es ist bereits bekannt, daß bestimmte Azolyloxycarbonsäureamide als Herbizide verwendet werden können (vgl. z. B. DE-A-2 822 155, DE-A-2 903 966, EP-A-5501 ; DE-A-2 914 003, DE-A-3 004 326, EP-A-18 497 und DE-A-3 038 636), z. B. 2-(2-Benzthiazolyl)-oxyessigsäure-N-methylanilid. Dieser Wirkstoff wird hauptsächlich zur Bekämpfung von Gramineen eingesetzt ; gegen einige wichtige Gräser und vor allem in niedrigeren Dosierungen ist diese Verbindung jedoch nicht immer voll wirksam. Ferner ist bekannt, daß bestimmte Aminoesterderivate von 3-Trihalomethyl-1,2,4-thiadiazolen eine herbizide Wirksamkeit besitzen sollen (vgl. US-A-4 228 290).

Es wurden nun neue substituierte 3-Trichlormethyl-1,2,4-thiadiazol-5-yl-oxyessigsäureamide der Formel

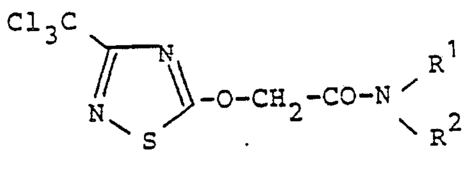

(I)

aufgefunden, in welcher

$R^1$ und $R^2$, welche gleich oder verschieden sein können, einzeln für Alkyl, Cyanoalkyl, Alkoxyalkyl, Alkylthioalkyl, Alkenyl, Alkinyl, Alkoxy, jeweils mit bis zu 10 C-Atomen, Cycloalkyl oder Cycloalkenyl mit jeweils bis zu 12 C-Atomen, Aralkyl mit 1 oder 2 C-Atomen im Alkylteil und 6 oder 10 C-Atomen im Arylteil, welcher gegebenenfalls durch Halogen substituiert ist, oder für Aryl mit 6 oder 10 C-Atomen stehen, wobei der Arylrest durch 1 bis 3 Halogenatome, 1 bis 3 Alkyl- oder Halogenalkylgruppen mit jeweils 1 bis 4 C-Atomen, durch Nitro, Cyano oder Alkoxy mit 1 bis 4 C-Atomen substituiert sein kann, oder worin die Reste $R^1$ und $R^2$ zusammen mit dem Stickstoffatom, an das sie gebunden sind, einen gegebenenfalls durch 1 bis 3 Alkylgruppen mit jeweils 1 bis 5 C-Atomen substituierten, teilweise ungesättigten und/oder benzanellierten Monocyclus oder Bicyclus mit bis zu 15 C-Atomen bilden, oder worin die Reste $R^1$ und $R^2$ zusammen mit dem Stickstoffatom, an das sie gebunden sind, einen gegebenenfalls durch 1 bis 3 Alkylgruppen mit jeweils 1 bis 5 C-Atomen substituierten, gesättigten und gegebenenfalls ein weiteres Stickstoffatom, Sauerstoffatom oder Schwefelatom enthaltenden Monocyclus mit bis zu 6 C-Atomen bilden.

Man erhält die neuen Verbindungen der Formel (I), wenn man Hydroxyessigsäureamide der Formel

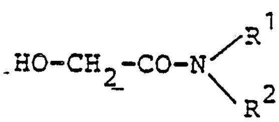

(II)

in welcher

$R^1$ und $R^2$ die oben angegebene Bedeutung haben,

mit 5-Chlor-3-trichlormethyl-1,2,4-thiadiazol der Formel

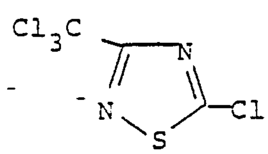

(III)

in Gegenwart eines Säureakzeptors und gegebenenfalls unter Verwendung eines Verdünnungsmittels umsetzt.

Die neuen substituierten 3-Trichlormethyl-1,2,4-thiadiazol-5-yl-oxyessigsäureamide der Formel (I) zeichnen sich durch starke herbizide Wirksamkeit aus.

Überraschenderweise sind die erfindungsgemäßen Wirkstoffe der Formel (I) gegen eine Reihe von wichtigen Gräsern deutlich besser wirksam als das vorbekannte 2-(2-Benzthiazolyl)-oxyessigsäure-N-methylanilid. Die neuen Wirkstoffe stellen somit eine Bereicherung der Technik dar.

Die Erfindung betrifft insbesondere Verbindungen der Formel (I), in welcher

$R^1$ für $C_1$-$C_5$-Alkyl, Cyanoethyl, $C_1$-$C_4$-Alkoxy-ethyl, Allyl, Propargyl, 1-Methyl-propargyl oder 1,1-Dimethyl-propargyl steht und

$R^2$ für $C_1$-$C_5$-Alkyl, $C_1$-$C_5$-Alkoxy, Cyanoethyl, $C_1$-$C_4$-Alkoxy-ethyl, Allyl, Propargyl, 1-Methyl-propargyl, 1,1-Dimethylpropargyl, Cyclopentyl, Cyclohexyl, 3,4,6-Trimethyl-cyclohexen-1-yl, Benzyl, Naphthyl oder Phenyl, welches gegebenenfalls durch 1 bis 3 Reste (Methyl, Chlor, Fluor, Trifluormethyl, Methoxy,

Methylthio, Trifluormethoxy und/oder Trifluormethylthio) substituiert ist, steht oder worin die Reste $R^1$ und $R^2$ zusammen mit dem Stickstoffatom, an das sie gebunden sind, für Pyrrolidyl, Monoalkyl- oder Dialkyl-pyrrolidyl mit 1 bis 3 Kohlenstoffatomen je Alkylgruppe, Morpholinyl oder Dialkylmorpholinyl mit 1 bis 3 Kohlenstoffatomen je Alkylgruppe, Piperidyl, Monoalkyl-, Dialkyl- oder Trialkylpiperidyl mit 1 bis 3 Kohlenstoffatomen je Alkylgruppe, für Perhydroazepinyl (Hexamethylenimino-Rest), für den Heptamethylenimino-Rest, für 1,2,3,4-Tetrahydroindolyl, für Monoalkyl- oder Dialkyl-tetrahydroindolyl mit bis zu 3 Kohlenstoffatomen je Alkylgruppe, für Perhydroindolyl, Monoalkyl- oder Dialkylperhydroindolyl mit 1 bis 3 Kohlenstoffatomen je Alkylgruppe, für 1,2,3,4-Tetrahydrochinolyl oder 1,2,3,4-Tetrahydro-iso-chinolyl, Monoalkyl- oder Dialkyl-1,2,3,4-tetrahydrochinolyl oder -iso-chinolyl mit 1 bis 3 Kohlenstoffatomen je Alkylgruppe, für Perhydrochinolyl oder Perhydro-iso-chinolyl, Monoalkyl- oder Dialkyl-perhydrochinolyl oder -perhydroisochinolyl mit 1 bis 3 Kohlenstoffatomen je Alkylgruppe stehen. Aus dieser Gruppe sind diejenigen Verbindungen der allgemeinen Formel (I), worin $R^1$ für verzweigtes oder unverzweigtes Alkyl mit 1-5 C-Atomen und $R^2$ für verzweigtes oder unverzweigtes Alkoxy mit 1-5 C-Atomen stehen, von ganz besonderem Interesse.

Verwendet man 5-Chlor-3-trichlormethyl-1,2,4-thiadiazol und beispielsweise Hydroxyessigsäure-N-methylanilid als Ausgangsstoffe, so kann der Reaktionsablauf nach dem erfindungsgemäßen Verfahren durch folgendes Formelschema wiedergegeben werden :

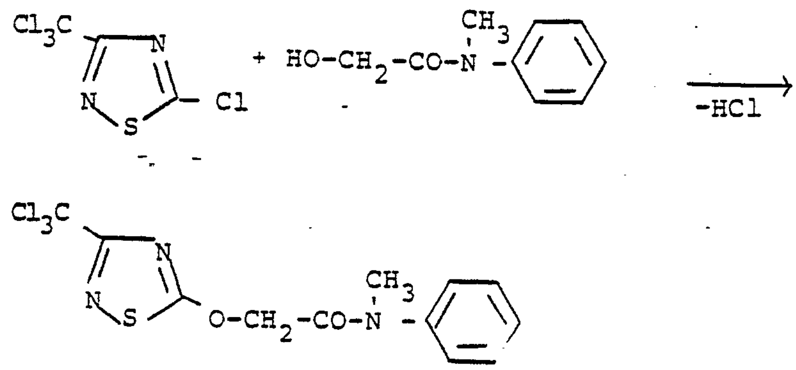


Die als Ausgangsstoffe zu verwendenden Hydroxyessigsäureamide sind durch Formel (II) definiert. In dieser Formel stehen $R^1$ und $R^2$ vorzugsweise bzw. insbesondere für diejenigen Reste, welche bereits im Rahmen der Substituentendefinitionen der Formel (I) vorzugsweise bzw. als insbesondere bevorzugt genannt sind.

Als Ausgangsstoffe der Formel (II) seien beispielsweise genannt :

Hydroxyessigsäure-dimethylamid, -diethylamid, -di-n-propylamid, -di-iso-propylamid, -N-methyl-N-iso-propylamid, -N-methyl-N-isobutyl-amid, -N-methyl-N-sek.-butylamid, -N-propyl-N-sek.-butyl-amid, -N-methyl-N-(2-cyanoethyl)-amid, -di-(2-methoxy-ethyl)-amid, -di-allyl-amid, -N-methyl-N-propargyl-amid, -N-methyl-N-(1-methyl-propargyl)-amid, -dipropargyl-amid, -N-methyl-N-cyclopentylamid, -N-methyl-N-cyclohexyl-amid, -N-methyl-N-(2-nitrophenyl)-, -N-methyl-N-(3-nitro-phenyl)-, -N-methyl-N-(4-nitro-phenyl)-amid, -N-methyl-N-(2-chlor-phenyl)-, -N-methyl-N-(3-chlor-phenyl)-, -N-methyl-N-(4-chlor-phenyl)-amid, -N-methyl-N-(3-nitro-6-methyl-phenyl)-amid, -N-ethyl-anilid, -N-ethyl-N-(2-nitro-phenyl)-, -N-ethyl-(3-nitro-phenyl)-, -N-ethyl-N-(4-nitro-phenyl)-amid, -N-ethyl-N-(2-chlor-phenyl)-, -N-ethyl-N-(3-chlor-phenyl)-, -N-ethyl-N-(4-chlor-phenyl)-amid, -N-ethyl-N-(3-nitro-6-methyl-phenyl)-amid, -N-propyl-anilid, -N-propyl-N-(2-nitro-phenyl)-, -N-propyl-N-(3-nitro-phenyl)-, -N-propyl-N-(4-nitro-phenyl)-amid, -N-propyl-N-(2-chlor-phenyl)-, -N-propyl-N-(3-chlor-phenyl)-, -N-propyl-N-(4-chlorphenyl)-amid, -N-propyl-N-(2-methyl-phenyl)-, -N-propyl-N-(3-methyl-phenyl)-, -N-propyl-N-(4-methyl-phenyl)-amid, -N-propyl-N-(3-nitro-6-methyl-phenyl)-amid, -N-butyl-anilid, -N-methyl-N-naphth(1)ylamid, -N-methyl-N-naphth(2)ylamid, -N-ethyl-N-naphth(1)ylamid, -N-ethyl-N-naphth(2)ylamid, -N-methyl-N-benzylamid, -N-ethyl-N-benzylamid, -N-propyl-N-benzylamid, -N-butyl-N-benzylamid, -pyrrolidid, -1-methyl-pyrrolidid, -morpholid, -piperidid, -2-methyl-piperidid, -2-methyl-piperidid, -4-methyl-piperidid, -2,4-dimethyl-piperidid, -3,5-dimethyl-piperidid, -3,5-Diethylpiperidid, -2,4,6-trimethyl-piperidid, -2-ethyl-piperidid, -4-ethyl-piperidid, -2,4-diethyl-piperidid, -2,4,6-triethylpiperidid, -2-methyl-4-ethyl-piperidid, -2, -ethyl-4-methyl-piperidid, -2-methyl-5-ethyl-piperidid, -2-ethyl-5-methyl-piperidid, -2-methyl-6-ethyl-piperidid, -1,2,3,4-tetrahydroindolid, -2-methyl-1,2,3,4-tetrahydroindolid, -perhydroindolid, -2-methyl-perhydroindolid, 2,2-dimethyl-perhydroindolid, -2-methyl-1,2,3,4-tetrahydrochinolid, -perhydrochinolid, -2-methyl-perhydrochinolid, -1,2,3,4-tetrahydro-isochinolid und -perhydroisochinolid ; ferner -N-methyl-N-(2-methylthio-phenyl)-, -N-methyl-N-(3-methylthio-phenyl)- und -N-methyl-N-(4-methylthio-phenyl)-amid, -N-methyl-N-(2-fluorphenyl)-, -N-methyl-N-(3-fluorphenyl)- und -N-methyl-N-(4-fluorphenyl)-amid ; -N-methyl-N-(2-trifluormethylphenyl)-, -N-methyl-N-(3-trifluormethylphenyl)- und -N-methyl-N-(4-trifluormethylphenyl)-amid ; -N-methyl-N-(2-trifluormethoxyphenyl)-, -N-methyl-N-(3-trifluormethoxyphenyl)- und -N-methyl-N-(4-trifluormethoxyphenyl)-amid ; -N-methyl-N-(2-trifluorethylphenyl)-amid.

Die Hydroxy-carbonsäureamide der Formel (II) sind teilweise bekannt (vgl. US-PS 3 399 988 ; DE-OS'en

2 201 432 und 2 647 481). Sie können, wie im nachstehenden Schema skizziert, ausgehend von Chloracetylchlorid hergestellt werden :

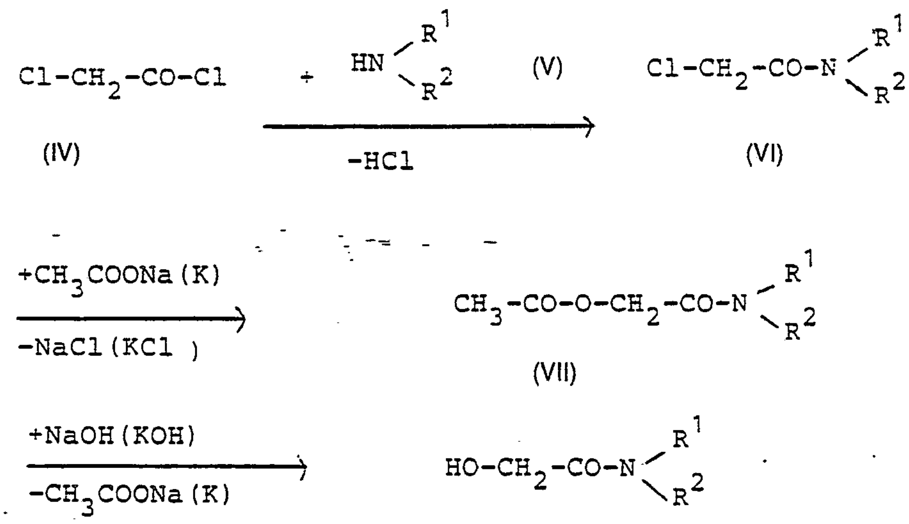


(IV) (V) (VI) (VII)

(II)

Hierzu wird zunächst das literaturbekannte Chloracetylchlorid der Formel (IV) mit Aminen der Formel (V) — wobei $R^1$ und $R^2$ die oben angegebene Bedeutung haben — gegebenenfalls in Gegenwart eines Säurebindemittels wie z. B. Triethylamin und gegebenenfalls unter Verwendung eines inerten Verdünnungsmittels wie z. B. 1,2-Dichlorethan, bei Temperaturen zwischen — 20 und 100 °C, vorzugsweise zwischen — 10 und 50 °C in die entsprechenden Chloressigsäureamide der Formel (VI) überführt. Die Aufarbeitung dieser Produkte erfolgt nach üblichen Methoden durch Waschen mit Wasser, Trocknen der organischen Phase und Abdestillieren des Lösungsmittels.

Die Verbindungen der Formel (VI) werden mit Natrium- oder Kalium-acetat, gegebenenfalls unter Verwendung eines Verdünnungsmittels wie z. B. Essigsäure oder Dimethylsulfoxid, bei Temperaturen zwischen 20 und 150 °C, vorzugsweise zwischen 50 und 120 °C, zu den entsprechenden Acetoxyessigsäureamiden der Formel (VII) umgesetzt. Soweit die Produkte hierbei kristallin anfallen, werden sie durch Absaugen isoliert. Andernfalls erfolgt die Aufarbeitung nach üblichen Methoden, beispielsweise durch Abdestillieren des Lösungsmittels im Vakuum, Aufnehmen des Rückstandes in Methylenchlorid, Waschen mit Wasser und Abdestillieren des Lösungsmittels.

Durch Umsetzung mit wäßrig-alkoholischer Natronlauge oder Kalilauge bei Temperaturen zwischen 0 und 100 °C, vorzugsweise zwischen 10 und 50 °C, können die Verbindungen der Formel (VII) zu den Verbindungen der Formel (II) entacyliert werden. Zur Isolierung der Produkte werden die Lösungsmittel im Vakuum abdestilliert, der Rückstand mit einem organischen Lösungsmittel wie z. B. Methylenchlorid oder Essigester extrahiert, die Lösung getrocknet und das Lösungsmittel abdestilliert.

Das als Ausgangsstoff zu verwendende 5-Chlor-3-trichlormethyl-1,2,4-thiadiazol ist durch die Formel (III) definiert. Diese Verbindung und ein Verfahren zu ihrer Herstellung sind bekannt (US-PS 3 260 588 (1966)).

Das Verfahren zur Herstellung der neuen Verbindungen der Formel (I) wird vorzugsweise unter Verwendung geeigneter Lösungs- oder Verdünnungsmittel durchgeführt. Als solche kommen praktisch alle inerten organischen Solventien in Frage. Hierzu gehören insbesondere Alkohole, wie Methanol, Ethanol, n- und iso-Propanol, n-, iso-, sek.- und tert.-Butanol, Ether wie Diethylether, Dibutylether, Tetrahydrofuran, Diglyme und Dioxan, Ketone wie Aceton, Methylethylketon, Methylisopropylketon und Methylisobutylketon, Nitrile wie Acetonitril und Propionsäurenitril, sowie die hochpolaren Lösungsmittel Dimethylformamid, Dimethylsulfoxid, Sulfolan und Hexamethylphosphorsäuretriamid.

Als Säureakzeptoren können praktisch alle üblicherweise verwendbaren Säurebindemittel eingesetzt werden : hierzu gehören insbesondere Alkali- und Erdalkalihydroxide bzw. -oxide wie Natrium-, Kaliumhydroxid und insbesondere Lithiumhydroxid sowie Calciumoxid oder Calcium-hydroxid, Alkali- und Erdalkali-carbonate wie Natrium-, Kalium- und Calciumcarbonat, Alkalialkoholate wie Natriummethylat, -ethylat und -tert.-butylat, Kaliummethylat, -ethylat und -tert.-butylat, ferner aliphatische, aromatische oder heterocyclische Amine wie Triethylamin, Dimethylanilin, Dimethylbenzylamin, Pyridin, Diazabicyclooctan und Diazabicycloundecen.

Die Reaktionstemperatur kann innerhalb eines größeren Bereichs variiert werden. Im allgemeinen arbeitet man zwischen — 50 und + 150 °C, vorzugsweise bei — 20 bis + 100 °C.

Das erfindungsgemäße Verfahren wird im allgemeinen bei Normaldruck durchgeführt.

Bei der Durchführung des erfindungsgemäßen Verfahrens setzt man auf 1 Mol 5-Chlor-3-trichlormethyl-1,2,4-thiadiazol der Formel (III) 1,0 bis 1,5 Mol Hydroxyessigsäureamid der Formel (II) ein. Die Umsetzung wird im allgemeinen in einem geeigneten Verdünnungsmittel durchgeführt und das Reaktionsgemisch wird bei der erforderlichen Temperatur mehrere Stunden gerührt.

Die Isolierung der Produkte erfolgt nach üblichen Methoden : Man destilliert gegebenenfalls einen Teil des Verdünnungsmittels unter vermindertem Druck ab und gießt den Rest der Reaktionsmischung in Wasser. Soweit die Produkte hierbei kristallin anfallen, werden sie durch Absaugen isoliert. Andernfalls werden die organischen Produkte mit einem mit Wasser nicht mischbaren Lösungsmittel wie z. B. Toluol oder Methylenchlorid extrahiert ; nach Waschen und Trocknen wird dann von der organischen Phase das Lösungsmittel im Vakuum abdestilliert. Die zurückleibenden Produkte werden durch ihren Schmelzpunkt bzw. ihren Brechungsindex charakterisiert.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z. B. bei den folgenden Pflanzen verwendet werden :

Dikotyle Unkräuter der Gattungen : Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dicotyle Kulturen der Gattungen : Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen : Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopercurus, Apera.

Monokotyle Kulturen der Gattungen : Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z. B. auf Industrie- und Gleisanlagen und auf Wegen und Plätzen mit und ohne Baumbewuchs. Ebenso können die Verbindungen zur Unkrautbekämpfung in Dauerkulturen z. B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe können bevorzugt als selektive Herbizide in verschiedenen dikotylen Kulturpflanzen, in Getreide und insbesondere in Reis eingesetzt werden.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoff-imprägnierte Natur- und synthetische Stoffe, Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage : Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, Alkohole, wie Butanol oder Glycol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage :

z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silicate ; als feste Trägerstoffe für Granulate kommen in Frage : z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel ; als Emulgier- und/oder schaumerzeugende Mittel kommen in Frage : z. B. nichtionogene und anionische Emulgatoren, wie Polyoxyethylen-Fettsäure-Ester, Polyoxyethylen-Fettalkohol-Ether, z. B. Alkylaryl-polyglykol-ether, Alkylsulfonate, Alkylsulfate, Arylsulfonate sowie Eiweißhydrolysate ; als Dispergiermittel kommen in Frage : z. B. Lignin-Sulfitablaugen und Methylcellulose.

Es können in den Formulierungen Haftmittel wie Carboxymethylcellulose, natürliche und syntheti-

sche pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azo-, Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierung oder Tankmischung möglich ist. Auch eine Mischung mit anderen bekannten Wirkstoffen, wie Fungiziden, Insektiziden, Akariziden, Nematiziden, Schutzstoffen gegen Vogelfraß, Wuchsstoffen, Pflanzennährstoffen und Bodenstrukturverbesserungsmitteln ist möglich.

Die Wirkstoffe können als solche, in Form ihrer Formulierungen oder der daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z. B. durch Gießen, Spritzen, Sprühen, Streuen oder Stäuben.

Die erfindungsgemäßen Wirkstoffe können sowohl vor als auch nach dem Auflaufen der Pflanzen appliziert werden.

Die Anwendung wird vorzugsweise vor dem Auflaufen der Pflanzen, also im pre-emergence-Verfahren, vorgenommen. Sie können auch vor der Saat in den Boden eingearbeitet werden.

Die aufgewandte Wirkstoffmenge kann in größeren Bereichen schwanken. Sie hängt im wesentlichen von der Art des gewünschten Effektes ab. Im allgemeinen liegen die Aufwandmengen zwischen 0,01 und 10 kg Wirkstoff pro ha, vorzugsweise zwischen 0,1 und 5 kg/ha.

Die nachfolgenden Beispiele dienen zur weiteren Erläuterung der Erfindung.

## Herstellungsbeispiele

### Beispiel 1

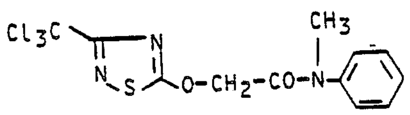

(1)

Eine Lösung von 8,4 g (0,21 Mol) Natriumhydroxid in 17 ml Wasser wird zu einer auf — 10 °C gekühlten Mischung aus 36 g (0,21 Mol) Hydroxyessigsäure-N-methylanilid, 50 g (0,21 Mol) 5-Chlor-3-trichlormethyl-1,2,4-thiadiazol und 300 ml Toluol unter Rühren tropfenweise gegeben. Das Reaktionsgemisch wird 20 Stunden bei — 10 °C gerührt. Nach Verrühren mit Wasser wird die organische Phase abgetrennt, mit Wasser gewaschen, getrocknet und eingeengt. Nach Digerieren mit Ligroin erhält man 65 g (84 % der Theorie) (3-Trichlormethyl-1,2,4-thiadiazol-5-yl)-yessigsäure-N-methylanilid in Form beigefarbener Kristalle vom Schmelzpunkt 69 °C.

### Beispiel 2

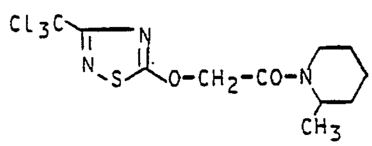

(2)

17 g (0,07 Mol) 5-Chlor-3-trichlormethyl-1,2,4-thiadiazol werden zu einer auf — 10 °C gekühlten Mischung aus 11 g (0,07 Mol) Hydroxyessigsäure-2-methylpiperidid, 4,5 g (0,07 Mol) Kaliumhydroxid-Pulver und 100 ml iso-Propanol unter Rühren gegeben. Das Reaktionsgemisch wird 20 Stunden bei — 10 °C gerührt, dann in Wasser gegossen, abgesaugt und mit Wasser gewaschen. Man erhält 18 g (72 % der Theorie) (3-Trichlormethyl-1,2,4-thiadiazol-5-yl)-oxyessigsäure-2-methylpiperidid in Form eines weißen Pulvers vom Schmelzpunkt 88 °C.

Analog zu den Beispielen 1 und 2 können die in der nachstehenden Tabelle 1 aufgeführten Verbindungen der Formel (I) erhalten werden :

(Siehe Tabelle 1 Seite 8 ff.)

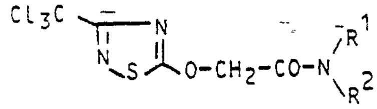

(I)

Tabelle 1

| Verbindung Nr. | 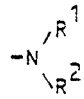 | Schmelzpunkt °C |
|---|---|---|
| 3 | $-N(CH_2-CH=CH_2)_2$ | 44 |
| 4 | $-N(CH_3)_2$ | 88 |
| 5 | 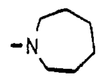 | 115 |
| 6 | 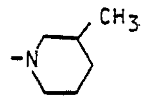 | 86 |
| 7 | 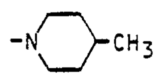 | 76 |
| 8 | 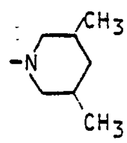 | 68 |
| 9 | $-N(C_2H_5)_2$ | 78 |
| 10 | 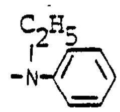 | 90 |
| 11 | 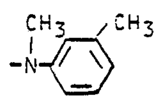 | 77 |
| 12 | 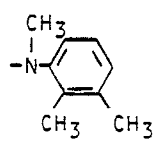 | 103 |
| 13 | 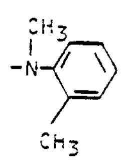 | 112 |
| 14 | 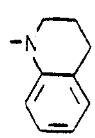 | 157 |
| 15 | 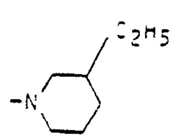 | 78 |

Tabelle 1 (Fortsetzung)

| Verbindung Nr. | 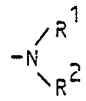 | Schmelzpunkt °C |
|---|---|---|
| 16 | 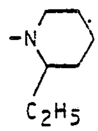 | 93 |
| 17 | 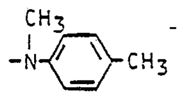 | 129 |
| 18 | 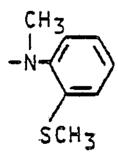 | |
| 19 | 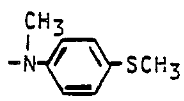 | |
| 20 | 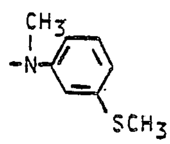 | |
| 21 | 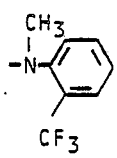 | |
| 22 | 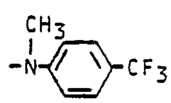 | |
| 23 | 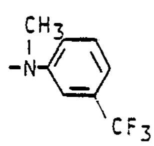 | |
| 24 | 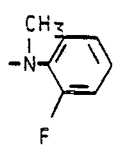 | |
| 25 | 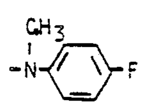 | |
| 26 | 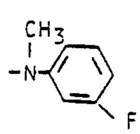 | |

Tabelle 1 (Fortsetzung)

| Verbindung Nr. | 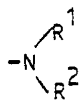 | Schmelzpunkt °C |
|---|---|---|
| 27 | 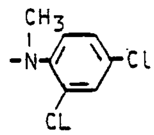 | |
| 28 | 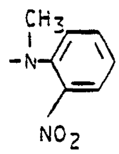 | |
| 29 | 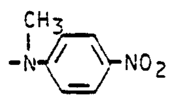 | |
| 30 | 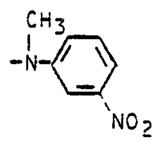 | |
| 31 | 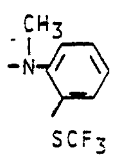 | |
| 32 | 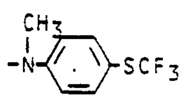 | |
| 33 | 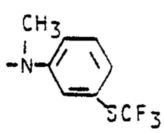 | |
| 34 | 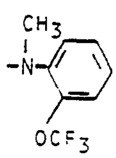 | |
| 35 | 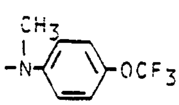 | |
| 36 | 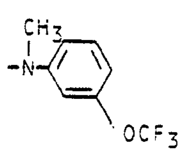 | |

Tabelle 1 (Fortsetzung)

| Verbindung Nr. | 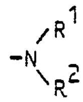 | Schmelzpunkt °C bzw. Brechungsindex |
|---|---|---|
| 37 | 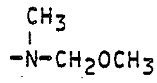 | |
| 38 | 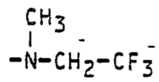 | |
| 39 | 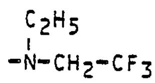 | |
| 40 | 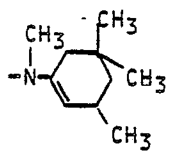 | |
| 41 | 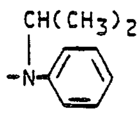 | |
| 42 | 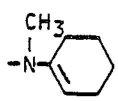 | 78 |
| 43 | 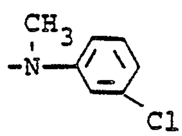 | 70 |
| 44 | 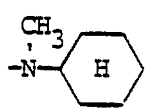 | 57 |
| 45 | $-N(C_4H_9-n)_2$ | $n_D^{20}$ : 1,5198 |
| 46 | $-N(C_3H_7-n)_2$ | $n_D^{20}$ : 1,5049 |
| 47 | 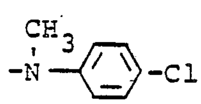 | 155 |
| 48 | 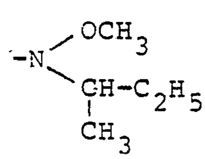 | $n_D^{20}$ : 1,5230 |

Beispiel A

Pre-emergence-Test

Lösungsmittel : 5 Gewichtsteile Aceton
Emulgator : 1 Gewichtsteil Alkylarylpolyglycolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten :

0 % = keine Wirkung (wie unbehandelte Kontrolle)
100 % = totale Vernichtung

In diesem Test zeigen z. B. die folgenden Verbindungen gemäß Herstellungsbeispielen eine ausgezeichnete Wirksamkeit : (1), (42), (44), (46), (48).

**Patentansprüche**

1. Substituierte 3-Trichlormethyl-1,2,4-thiadiazol-5-yl-oxyessigsäureamide der Formel

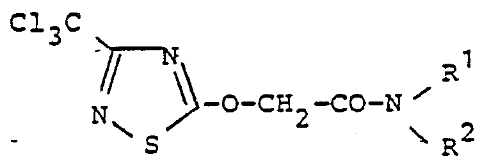

(I)

in welcher

$R^1$ und $R^2$, welche gleich oder verschieden sein können, einzeln für Alkyl, Cyanoalkyl, Alkoxyalkyl, Alkylthioalkyl, Alkenyl, Alkinyl, Alkoxy, jeweils mit bis zu 10 C-Atomen, Cycloalkyl oder Cyclo-alkenyl mit jeweils bis zu 12 C-Atomen, Aralkyl mit 1 oder 2 C-Atomen im Alkylteil und 6 oder 10 C-Atomen im Arylteil, welcher gegebenenfalls durch Halogen substituiert ist, oder für Aryl mit 6 oder 10 C-Atomen stehen, wobei der Arylrest durch 1 bis 3 Halogenatome, 1 bis 3 Alkyl- oder Halogenalkylgruppen mit jeweils 1 bis 4 C-Atomen, durch Nitro, Cyano oder Alkoxy mit 1 bis 4 C-Atomen substituiert sein kann, oder worin die Reste $R^1$ und $R^2$ zusammen mit dem Stickstoffatom, an das sie gebunden sind, einen gegebenenfalls durch 1 bis 3 Alkylgruppen mit jeweils 1 bis 5 C-Atomen substituierten, teilweise ungesättigten und/oder benzanellierten Monocyclus oder Bicyclus mit bis zu 15 C-Atomen bilden, oder worin die Reste $R^1$ und $R^2$ zusammen mit dem Stickstoffatom, an das sie gebunden sind, einen gegebenenfalls durch 1 bis 3 Alkylgruppen mit jeweils 1 bis 5 C-Atomen substituierten, gesättigten und gegebenenfalls ein weiteres Stickstoffatom, Sauerstoffatom oder Schwefelatom enthaltenden Monocyclus mit bis zu 6 C-Atomen bilden.

2. Verbindungen der Formel (I) gemäß Anspruch 1, dadurch gekennzeichnet, daß

$R^1$ für $C_1$-$C_5$-Alkyl, Cyanoethyl, $C_1$-$C_4$-Alkoxy-ethyl, Allyl, Propargyl, 1-Methyl-propargyl oder 1,1-Dimethyl-propargyl steht und

$R^2$ für $C_1$-$C_5$-Alkyl, $C_1$-$C_5$-Alkoxy, Cyanoethyl, $C_1$-$C_4$-Alkoxy-ethyl, Allyl, Propargyl, 1-Methyl-propargyl, 1,1-Dimethylpropargyl, Cyclopentyl, Cyclohexyl, 3,4,6-Trimethyl-cyclohexen-1-yl, Benzyl, Naphthyl oder Phenyl, welches gegebenenfalls durch 1 bis 3 Reste (Methyl, Chlor, Fluor, Trifluormethyl, Methoxy, Methylthio, Trifluormethoxy und/oder Trifluormethylthio) substituiert ist, steht

oder worin die Reste $R^1$ und $R^2$ zusammen mit dem Stickstoffatom, an das sie gebunden sind, für Pyrrolidyl, Mono-alkyl- oder Dialkyl-pyrrolidyl mit 1 bis 3 Kohlenstoffatomen je Alkylgruppe, Morpholinyl oder Dialkylmorpholinyl mit 1 bis 3 Kohlenstoffatomen je Alkylgruppe, Piperidyl, Monoalkyl-, Dialkyl- oder Trialkylpiperidyl mit 1 bis 3 Kohlenstoffatomen je Alkylgruppe, für Perhydroazepinyl (Hexamethylenimino-Rest), für den Heptamethylenimino-Rest, für 1,2,3,4-Tetrahydroindolyl, für Monoalkyl- oder Dialkyltetrahydroindolyl mit bis zu 3 Kohlenstoffatomen je Alkylgruppe, für Perhydroindolyl, Monoalkyl- oder Dialkylperhydroindolyl mit 1 bis 3 Kohlenstoffatomen je Alkylgruppe, für 1,2,3,4-Tetrahydrochinolyl oder 1,2,3,4-Tetrahydro-iso-chinolyl, Monoalkyl- oder Di-alkyl-1,2,3,4-tetrahydrochinolyl oder -iso-chinolyl mit 1 bis 3 Kohlenstoffatomen je Alkylgruppe, für Perhydrochinolyl oder Perhydro-iso-chinolyl, Monoalkyl- oder Dialkyl-perhydrochinolyl oder -perhydroisochinolyl mit 1 bis 3 Kohlenstoffatomen je Alkylgruppe stehen.

3. Verbindungen der Formel (I) gemäß Anspruch 1, dadurch gekennzeichnet, daß

$R^1$ für verzweigtes oder unverzweigtes Alkyl mit 1-5C-Atomen und
$R^2$ für verzweigtes oder unverzweigtes Alkoxy mit 1-5C-Atomen steht.

4. (3-Trichlormethyl-1,2,4-thiadiazol-5-yl)-oxyessigsäure-N-methoxy-N-isobutylamid der Formel

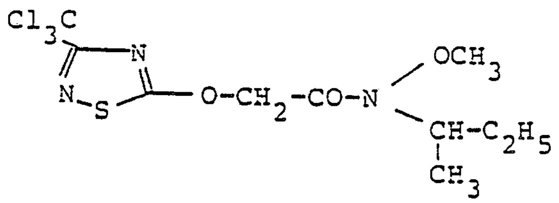

gemäß Anspruch 1.

5. (3-Trichlormethyl-1,2,4-thiadiazol-5-yl)-oxyessigsäure-N-methylanilid der Formel

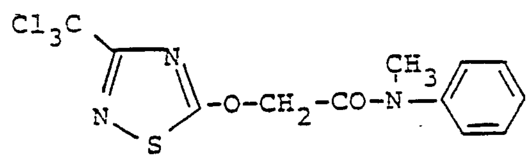

gemäß Anspruch 1.

6. (3-Trichlormethyl-1,2,4-thiadiazol-5-yl)-oxyessigsäure-N-methyl-N-(2.2.2-trifluorethyl)-amid und (3-Trichlormethyl-1,2,4-thiadiazol-5-yl)-oxyessigsäure-N-ethyl-N-(2.2.2-trifluorethyl)-amid.

7. Verfahren zur Herstellung von substituierten 3-Trichlormethyl-1,2,4-thiadiazol-5-yl-oxyessigsäureamiden der Formel (I) gemäß Anspruch 1, dadurch gekennzeichnet, daß man Hydroxyessigsäureamide der Formel

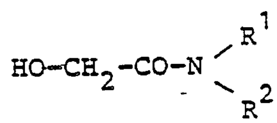

(II)

in welcher
$R^1$ und $R^2$ die oben angegebene Bedeutung haben,
mit 5-Chlor-3-trichlormethyl-1,2,4-thiadiazol der Formel

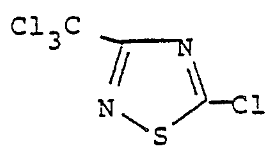

(III)

in Gegenwart eines Säureakzeptors und gegebenenfalls unter Verwendung eines Verdünnungsmittels umsetzt.

8. Herbizide Mittel, gekennzeichnet durch einen Gehalt an substituierten 3-Trichlormethyl-1,2,4-thiadiazol-5-yl-oxyessigsäureamiden der Formel (I) gemäß Anspruch 1.

9. Verwendung von substituierten 3-Trichlormethyl-1,2,4-thiadiazol-5-yl-oxyessigsäureamiden der Formel (I) gemäß Anspruch 1 zur Bekämpfung von Pflanzenwachstum.

10. Verfahren zur Herstellung von herbiziden Mitteln, dadurch gekennzeichnet, daß man substituierte 3-Trichlormethyl-1,2,4-thiadiazol-5-yl-oxyessigsäureamide der Formel (I) gemäß Anspruch 1 mit Streckmitteln und/oder oberflächenaktiven Mitteln vermischt.

**Claims**

1. Substituted 3-trichloromethyl-1,2,4-thiadiazol-5-yl-oxyacetic acid amides of the formula

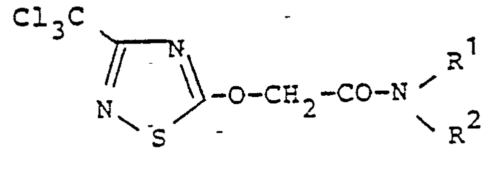

(I)

in which
$R^1$ and $R^2$, which can be identical or different, individually represent alkyl, cyanoalkyl, alkoxyalkyl, alkylthioalkyl, alkenyl, alkinyl, alkoxy, each with up to 10 C atoms, cycloalkyl or cycloalkenyl, each with up to 12 C atoms, aralkyl with 1 or 2 C atoms in the alkyl part and 6 or 10 C atoms in the aryl part, which is

optionally substituted by halogen, or aryl with 6 or 10 C atoms, it being possible for the aryl radical to be substituted by 1 to 3 halogen atoms, 1 to 3 alkyl or halogenoalkyl groups, each with 1 to 4 C atoms, by nitro, cyano or alkoxy with 1 to 4 C atoms, or wherein the radicals $R^1$ and $R^2$, together with the nitrogen atoms to which they are bonded, form a partially unsaturated and/or benzo-fused monocyclic or bicyclic structure which has up to 15 C atoms and is optionally substituted by 1 to 3 alkyl groups, each with 1 to 5 C atoms, or wherein the radicals $R^1$ and $R^2$, together with the nitrogen atom to which they are bonded, form a saturated monocyclic structure which has up to 6 C atoms, is optionally substituted by 1 to 3 alkyl groups, each with 1 to 5 C atoms, and optionally contains a further nitrogen atom, oxygen atom or sulphur atom.

2. Compounds of the formula (I) according to Claim 1, characterised in that

$R^1$ represents $C_1$-$C_5$-alkyl, cyanoethyl, $C_1$-$C_4$-alkoxy-ethyl, allyl, propargyl, 1-methyl-propargyl or 1,1-dimethyl-propargyl and

$R^2$ represents $C_1$-$C_5$-alkyl, $C_1$-$C_5$-alkoxy, cyanoethyl, $C_1$-$C_4$-alkoxy-ethyl, allyl, propargyl, 1-methyl-propargyl, 1,1-dimethylpropargyl, cyclopentyl, cyclohexyl, 3,4,6-trimethyl-cyclohexen-1-yl, benzyl, naphthyl or phenyl, which is optionally substituted by 1 to 3 radicals (methyl, chlorine, fluorine, trifluoromethyl, methoxy, methylthio, trifluoromethoxy and/or trifluoromethylthio),

or wherein the radicals $R^1$ and $R^2$, together with the nitrogen atom to which they are bonded, represent pyrrolidyl, monoalkyl- or dialkyl-pyrrolidyl with 1 to 3 carbon atoms per alkyl group, morpholinyl or dialkyl-morpholinyl with 1 to 3 carbon atoms per alkyl group, piperidyl, monoalkyl-, dialkyl- or trialkylpiperidyl with 1 to 3 carbon atoms per alkyl group, perhydroazepinyl (hexamethyleneimino radical), the heptamethyleneimino radical, 1,2,3,4-tetrahydroindolyl, monoalkyl- or dialkyl-tetrahydroindolyl with up to 3 carbon atoms per alkyl group, perhydroindolyl, monoalkyl or dialkylperhydroindolyl with 1 to 3 carbon atoms per alkyl group, 1,2,3,4-tetrahydroquinolyl or 1,2,3,4-tetrahydro-iso-quinolyl, monoalkyl- or dialkyl-1,2,3,4-tetrahydroquinolyl or -iso-quinolyl with 1 to 3 carbon atoms per alkyl group, perhydroquinolyl or perhydro-iso-quinolyl, monoalkyl- or dialkyl-perhydroquinolyl or -perhydro-isoquinolyl with 1 to 3 carbon atoms per alkyl group.

3. Compounds of the formula (I) according to Claim 1, characterised in that

$R^1$ represents branched or unbranched alkyl with 1-5 C atoms and

$R^2$ represents branched or unbranched alkoxy with 1-5 C atoms.

4. (3-Trichloromethyl-1,2,4-thiadiazol-5-yl)-oxyacetic acid N-methoxy-N-isobutylamide of the formula

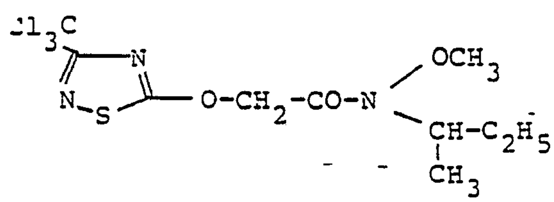

according to Claim 1.

5. (3-Trichloromethyl-1,2,4-thiadiazol-5-yl)-oxyacetic acid N-methylanilide of the formula

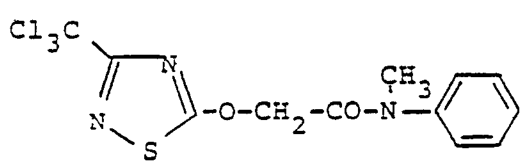

according to Claim 1.

6. (3-Trichloromethyl-1,2,4-thiadiazol-5-yl)-oxyacetic acid N-methyl-N-(2.2.2-trifluoroethyl)-amide and (3-trichloromethyl-1,2,4-thiadiazol-5-yl)-oxyacetic acid N-ethyl-N-(2.2.2-trifluoroethyl)-amide.

7. Process for the preparation of substituted 3-trichloromethyl-1,2,4-thiadiazol-5-yl-oxyacetic acid amides of the formula (I) according to Claim 1, characterised in that hydroxyacetic acid amides of the formula

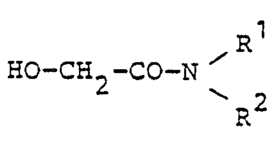

(II)

in which

$R^1$ and $R^2$ have the abovementioned meaning, are reacted with 5-chloro-3-trichloromethyl-1,2,4-thiadiazole of the formula

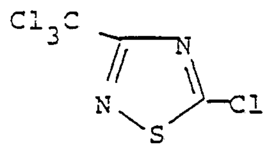

(III)

in the presence of an acid acceptor and if appropriate using a diluent.

8. Herbicidal agents, characterised in that they contain substituted 3-trichloromethyl-1,2,4-thiadiazol-5-yl-oxyacetic acid amides of the formula (I) according to Claim 1.

9. Use of substituted 3-trichloromethyl-1,2,4-thiadiazol-5-yl-oxyacetic acid amides of the formula (I) according to Claim 1 for combating plant growth.

10. Process for the preparation of herbicidal agents, characterised in that substituted 3-trichloromethyl-1,2,4-thiadiazol-5-yl-oxyacetic acid amides of the formula (I) according to Claim 1 are mixed with extenders and/or surface-active agents.

**Revendications**

1. 3-trichlorométhyl-1,2,4-thiadiazole-5-yl-oxyacétamides substitués de formule :

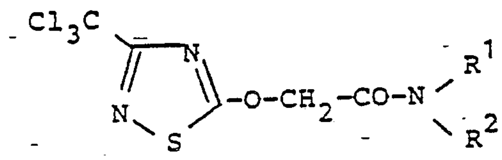

(I)

dans laquelle :

$R^1$ et $R^2$, qui peuvent être identiques ou différents, représentent chacun un reste alkyle, cyanoalkyle, alcoxyalkyle, alkylthioalkyle, alcényle, alcynyle, alcoxy, ayant chacun jusqu'à 10 atomes de carbone, cycloalkyle ou cycloalcényle ayant chacun jusqu'à 12 atomes de carbone, aralkyle ayant 1 ou 2 atomes de carbone dans la partie alkyle et 6 ou 10 atomes de carbone dans la partie aryle, qui peut éventuellement être substitué par de l'halogène, ou bien un reste aryle comportant 6 ou 10 atomes de carbone, le reste aryle pouvant être substitué par 1 à 3 atomes d'halogène, par 1 à 3 groupes alkyle ou halogénoalkyle ayant chacun 1 à 4 atomes de carbone, par un groupe nitro, cyano ou alcoxy ayant 1 à 4 atomes de carbone, ou bien les restes $R^1$ et $R^2$, pris avec l'atome d'azote auquel ils sont fixés, forment un groupe monocyclique ou bicyclique comprenant jusqu'à 15 atomes de carbone, partiellement insaturé et/ou comportant un noyau benzénique condensé, éventuellement substitué par 1 à 3 groupes alkyle ayant chacun 1 à 5 atomes de carbone, ou bien les restes $R^1$ et $R^2$ forment, avec l'atome d'azote auquel ils sont fixés, un groupe monocyclique ayant jusqu'à 6 atomes de carbone, saturé, éventuellement substitué par 1 à 3 groupes alkyle ayant chacun 1 à 5 atomes de carbone, et contenant éventuellement un autre atome d'azote, un autre atome d'oxygène ou un autre atome de soufre.

2. Composés de formule (I) selon la revendication 1, caractérisés en ce que $R^1$ représente un reste alkyle en $C_1$-$C_5$, cyanoéthyle, alcoxy (en $C_1$-$C_4$)-éthyle, allyle, propargyle, 1-méthylpropargyle ou 1,1-diméthylpropargyle, et

$R^2$ représente un reste alkyle en $C_1$-$C_5$, alcoxy en $C_1$-$C_5$, cyanoéthyle, alcoxy (en $C_1$-$C_4$)-éthyle, allyle, propargyle, 1-méthylpropargyle, 1,1-diméthylpropargyle, cyclopentyle, cyclohexyle, 3,4,6-triméthylcyclohexène-1-yle, benzyle, naphtyle ou phényle, qui est éventuellement substitué par 1 à 3 restes (méthyle, chloro, fluoro, trifluorométhyle, méthoxy, méthylthio, trifluorométhoxy et/ou trifluorométhylthio),

ou bien les restes $R^1$ et $R^2$ forment, avec l'atome d'azote auquel ils sont fixés, un groupe pyrrolidyle, monoalkylpyrrolidyle ou dialkylpyrrolidyle comportant 1 à 3 atomes de carbone dans chaque groupe alkyle, morpholinyle ou dialkylmorpholinyle, ayant 1 à 3 atomes de carbone par groupe alkyle, pipéridyle, monoalkylpipéridyle, dialkylpipéridyle ou trialkylpipéridyle, comportant 1 à 3 atomes de carbone dans chaque groupe alkyle, perhydroazépinyle (reste hexaméthylèneimino), le reste heptaméthylèneimino, le reste 1,2,3,4-tétrahydroindolyle, un reste monoalkyl- ou dialkyltétrahydroindolyle comportant jusqu'à 3 atomes de carbone dans chaque groupe alkyle, un reste perhydroindolyle monoalkyl- ou dialkylperhydroindolyle comportant 1 à 3 atomes de carbone dans chaque groupe alkyle, un reste 1,2,3,4-tétrahydroquinolyle ou 1,2,3,4-tétrahydro-isoquinolyle, monoalkyl- ou dialkyl-1,2,3,4-tétrahydroquinolyle ou -isoquinolyle comportant 1 à 3 atomes de carbone dans chaque groupe alkyle, un reste perhydroquinolyle ou perhydroisoquinolyle, un reste monoalkyl- ou dialkylperhydroquinolyle ou un -perhydroisoquinolyle comportant 1 à 3 atomes de carbone par groupe alkyle.

3. Composés de formule (I) selon la revendication 1, caractérisés en ce que :

$R^1$ représente un reste alkyle ramifié ou non ramifié ayant 1 à 5 atomes de carbone, et

$R^2$ représente un reste alcoxy, ramifié ou non ramifié, ayant 1 à 5 atomes de carbone.

4. N-méthoxy-N-isobutylamide de l'acide (3-trichlorométhyl-1,2,4-thiadiazole-5-yl)-oxyacétique de formule :

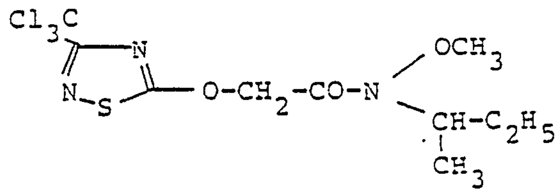

selon la revendication 1.

5. N-méthylanilide de l'acide (3-trichlorométhyl-1,2,4-thiadiazole-5-yle)-oxyacétique de formule :

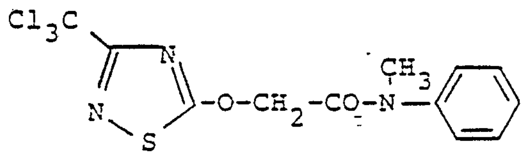

selon la revendication 1.

6. N-méthyl-N-(2,2,2-trifluoroéthyl)-amide de l'acide (3-trichlorométhyl-1,2,4-thiadiazole-5-yl)-oxyacétique et N-(2,2,2-trifluoréthyl)-amide de l'acide (3-trichlorométhyl-1,2,4-thiadiazole-5-yl)-oxyacétique.

7. Procédé pour préparer des 3-trichlorométhyl-1,2,4-thiadiazole-5-yl-oxyacétamides de formule (I) selon la revendication 1, caractérisé en ce qu'on fait réagir des hydroxyacétamides de formule :

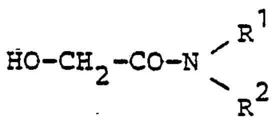

(II)

dans laquelle $R^1$ et $R^2$ ont le sens indiqué ci-dessus, avec le 5-chloro-3-trichlorométhyl-1,2,4-thiadiazole de formule :

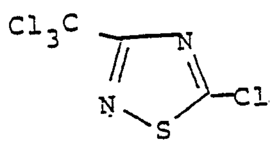

(III)

en présence d'un accepteur d'acide et éventuellement en utilisant un diluant.

8. Produits herbicides, caractérisés par une teneur en des 3-trichlorométhyl-1,2,4-thiadiazole-5-yl-oxyacétamides substitués de formule (I) selon la revendication 1.

9. Utilisation de 3-trichlorométhyl-1,2,4-thiadiazole-5-yl-oxyacétamides substitués de formule (I) selon la revendication 1, pour combattre la croissance de plantes.

10. Procédé de préparation de produits herbicides, caractérisé en ce qu'on mélange des 3-trichlorométhyl-1,2,4-thiadiazole-5-yl-oxyacétamides substitués, de formule (I) selon la revendication 1, avec des agents d'allongement et/ou des agents tensioactifs.